# EUROPEAN PATENT SPECIFICATION

(11) **EP 1 482 793 B1**
(45) Date of publication and mention of the grant of the patent: **31.12.2008**
(21) Application number: 03713993.8
(22) Date of filing: 07.03.2003
(51) Int. Cl.: A01N 37/18, C07K 1/00, C07H 21/02

(54) **EFFECT OF TREATMENT WITH 4,5-DIHYDROXY- 2-CYCLOPENTEN-1-ONE (DHCP) ON GENE EXPRESSION AND QUORUM-SENSING IN BACTERIA**
WIRKUNG DER BEHANDLUNG MIT 4,5-DIHYDROXY- 2-CYCLOPENTEN-1-ON (DHCP) AUF DIE GENEXPRESSION UND QUORUM-MESSUNG IN BAKTERIEN
EFFET D'UN TRAITEMENT AU 4,5-DIHYDROXY- 2-CYCLOPENTENE-1-ONE (DHCP) SUR L'EXPRESSION GENIQUE ET LA DENSITE BACTERIENNE (QUORUM SENSING)

(30) Priority: 13.03.2002 US 363548 P; 09.08.2002 US 402041 P
(43) Date of publication of application: 08.12.2004
(60) Divisional application: 08011067.9
(73) Proprietor: Takara Bio, Inc., Otsu-shi, Shiga 520-2193 (JP)
(72) Inventor: PHADTARE, Sangita, Highland Park, NJ 08904 (US); KATO, Ikunoshin, Shiga 529-1851 (JP); INOUYE, Masayori, The Colony House, New Brunswick, NJ 08901 (US)
(74) Representative: Schnappauf, Georg
(86) International application number: PCT/US2003/007081
(87) International publication number: WO 2003/077844

(56) References cited:
- EP-A- 0 941 981
- WO-A-02/17902
- US-B1- 6 559 176
- SCHAUDER STEPHAN ET AL: "The LuxS family of bacterial autoinducers: Biosynthesis of a novel quorum-sensing signal molecule" MOLECULAR MICROBIOLOGY, vol. 41, no. 2, July 2001 (2001-07), pages 463-476, XP002329742 ISSN: 0950-382X
- PHADTARE S ET AL: "ANTIBACTERIAL ACTIVITY OF 4,5-DIHYDROXY-2-CYCLOPENTAN-1-ONE (DHCP) AND CLONING OF A GENE CONFERRING DHCP RESISTANCE IN ESCHERICHIA COLI" JOURNAL OF MOLECULAR MICROBIOLOGY AND BIOTECHNOLOGY, HORIZON SCIENTIFIC PRESS, WYMONDHAM,, GB, vol. 3, no. 3, 2001, pages 461-465, XP009035099 ISSN: 1464-1801
- PHADTARE SANGITA ET AL: "DNA microarray analysis of the expression profile of Escherichia coli in response to treatment with 4,5-dihydroxy-2-cyclopenten-1-one" JOURNAL OF BACTERIOLOGY, vol. 184, no. 23, December 2002 (2002-12), pages 6725-6729, XP002329743 ISSN: 0021-9193
- DATABASE MEDLINE [Online] US NATIONAL LIBRARY OF MEDICINE, (BETHESDA, MD, USA) 03 July 2001 PHADTARE ET AL.: 'Antibacterial activity of DHCP and cloning of a gene conferring DHCP resistance in E. coli', XP002968949 Database accession no. 2001274440

### SEQUENCE LISTING

<110> Takara Bio, Inc.
<120> EFFECT OF TREATMENT WITH 4,5-DIHYDROXY-2-CYCLOPENTEN-1-ONE (DHCP) ON GENE EXPRESSION AND QUORUM-SENSING IN BACTERIA
<130> 38-77
<140> EP 03713993.8
   <141> 2003-03-07
<150> 60/363,548
   <151> 2002-03-13
<150> 60/402,041
   <151> 2002-08-09
<160> 8
<170> PatentIn version 3.2
<210> 1
   <211> 24
   <212> DNA
   <213> Artificial Sequence
<220>
   <223> Synthetic
<400> 1
   tacagccagc agagttttcg aaat 24
<210> 2
   <211> 24
   <212> DNA
   <213> Artificial Sequence
<220>
   <223> Synthetic
<400> 2
   tacagccagc agagttttcg aaat 24
<210> 3
   <211> 24
   <212> DNA
   <213> Artificial Sequence
<220>
   <223> Synthetic
<400> 3
   cagcgtattc tgactcataa ggtg 24
<210> 4
   <211> 24
   <212> DNA
   <213> Artificial Sequence
<220>
   <223> Synthetic
<400> 4
   agtggctgtg gtgttatgga tatc 24
<210> 5
   <211> 24
   <212> DNA
   <213> Artificial Sequence
<220>
   <223> Synthetic
<400> 5
   caggcgaacc agcggcgtgt gacc 24
<210> 6
   <211> 24
   <212> DNA
   <213> Artificial Sequence
<220>
   <223> Synthetic
<400> 6
   caggcgaacc agcggcgtgt gacc 24
<210> 7
   <211> 23
   <212> DNA
   <213> Artificial Sequence
<220>
   <223> Synthetic
<400> 7
gattaatatc agacgcaaat cct 23
<210> 8
   <211> 26
   <212> DNA
   <213> Artificial Sequence
<220>
   <223> Synthetic
<400> 8
   acgggatcct tcatcattat ttatta 26

## Description

### BACKGROUND OF THE INVENTION

### Field of the Invention

Regulation of gene expression and quorum-sensing in bacteria.

### Description of the Related Art

A number of antibiotics are available against a variety of bacteria; however, in recent years, the emergence of multiple drug resistant bacteria has been a main concern. This has provided an incentive to search for newer and more effective antibacterial compounds. Previously, the laboratory of the present inventors reported one such compound, 4,5-dihydroxy-2-cyclopenten-1-one (DHCP), having antibacterial activity against a variety of gram-negative and gram-positive bacteria, such as *E. coli, Salmonella, Bacillus, Staphylococcus,* etc. DHCP is prepared by the heat-treatment of uronic acid or its derivatives (Koyama et al., 1999). It is also produced from roasted or parched vegetables, fruits, cereals, mushrooms, sea algae, cortex or cartilage. It has been shown to have potential applications as therapeutic or preventive agent against cancer and also as an antibacterial agent in antiseptics, dentrifices, cosmetics and bathing agents (Koyama et al., 1999). It is possible that DHCP is also synthesized by microorganisms. When S-adenosylmethionine (SAM) is used as methyl donor in the biosynthesis of DNA, RNA or proteins, several SAM-dependent methyltransferases transfer the methyl group from SAM to its substrates producing S-adenosylhomocysteine (SAH), which is then degraded via the enzyme Pfs to produce S-ribosylhomocysteine (SRH). This is finally converted to homocysteine and 4,5-dihydroxy-2,3-pentanedione (Miller et al., 1968 and Schauder et al., 2001). Based on the structure, it is possible that DHCP is formed from the latter.

A multicopy suppressor for the DHCP toxicity was isolated from an *E. coli* genomic library. The gene encoding this suppressor was designated as *dep* and the putative protein encoded by this gene as Dep. The Dep protein showed high homology to known efflux proteins conferring resistance to a number of antibiotics including chloramphenicol, bicyclomycin and tetracycline. However, it did not confer cross-resistance to any of the antibiotics tested (Phadtare et al., 2001). The exact mechanism of action of DHCP is not known.

Recently, DHCP has also been of interest in the studies regarding quorum-sensing in *E. coli* (Schauder et al., 2001). Many bacteria have the ability to control expression of specific genes by secreting low-molecular-weight signaling phermones in association with the growth phase. This process is termed as quorum-sensing. Physiological processes controlled by quorum-sensing occur in diverse species of bacteria and include bioluminescence, antibiotic synthesis, pathogenicity, protein secretion, capsular exopolysaccharide synthesis, biofilm formation and motility (for review see Miller et al., 2001; Schauder et al., 2001 and Whitehead et al., 2001). The study of quorum-sensing is especially important as controlling cell density is one of its prominent features and many compounds generating such a response have antibacterial activity. *Vibrio harveyi,* a gram-negative bioluminescent marine bacterium regulates light production in response to two distinct autoinducers AI-1 and AI-2. AI-1 is homoserine lactone and is used for intraspecies communication. AI-2 is used for interspecies communication and its structure is not known. AI-2 is produced from SAM and the biosynthetic pathway and the biochemical intermediates in its biosynthesis are identical in *E. coli, S. typhimurium, V. harveyi, V. cholerae* and *Enterococcus faecalis.* Several compounds were screened as candidates for AI-2, with DHCP being one of them. However, DHCP did not show any activity in the screening assay and was thus excluded as a possible candidate for AI-2 (Schauder et al., 2001).

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention provides a method for regulating a gene in a microorganism by contacting a microorganism, in which the gene is to be regulated, with 4,5-dihydroxy-2-cyclopenten-1-one (DHCP) and thereby regulating the gene in the microorganism. The gene to be regulated includes genes responding to stress, genes involved in membrane synthesis and membrane function, genes encoding proteins with diverse functions, and genes encoding proteins of unknown function. Non-limiting examples of such a gene to be regulated include the genes listed in Tables 1-7 hereinbelow and homologues thereof. Preferably, the gene is involved in quorum-sensing processes such as a result of switching on/off of a quorum-sensing circuit or of an activity of an interspecies autoinducer AI-2.

The present invention also provides a method for screening a physiologically active substance, which influences the expression level of a gene whose expression level is also influenced by DHCP, and a physiologically active substance obtainable by this method. This method involves incubating a microorganism in the presence and in the absence of the candidate substance and then determining the expression level of genes in the microorganism in the presence and in the absence of the candidate substance, where the genes are ones in which their expression levels are influenced by DHCP. A candidate substance is identified as a physiologically active substance which significantly influences the expression level of at least one of the genes if the expression levels in the presence of the candidate substance is significantly enhanced or reduced relative to expression levels in the absence of the candidate substance. Non-limiting examples of a gene for use in this screening method include the genes listed in Tables 1-7 hereinbelow and homologues thereof. As preferred embodiments, the expression levels of the genes are determined based on the amounts of mRNA transcribed from the genes or by hybridization using a DNA microarray.

The present invention is directed to a method for enhancing the production of a recombinant polypeptide in a microorganism involving culturing a microorganism in the presence of DHCP to enhance the expression and production of a recombinant polypeptide and then recovering the recombinant polypeptide produced from the culture.

The present invention provides a method for inhibiting the activity of an interspecies quorum-sensing inducer, such as inducer AI-2, using DHCP as an active ingredient.

Still further, the present invention provides the use of DHCP for the manufacture of a composition for regulating a gene in a microorganism by a method according to the present invention and to compositions containing DHCP as an active ingredient for regulating a gene in a microorganism; for regulating quorum-sensing, such as the expression of cysK; for switching on/off of a quorum-sensing circuit; for inhibiting an activity of an interspecies quorum-sensing inducer, such as AI-2; for regulating expression of a gene responding to stress; for regulating expression of a regulatory gene for quorum-sensing; for promoting secretion of a recombinant protein; for regulating expression of a gene selected from those listed in Tables 1 to 7, or for maintaining homeostasis.

### BRIEF DESCRIPTION OF THE DRAWINGS

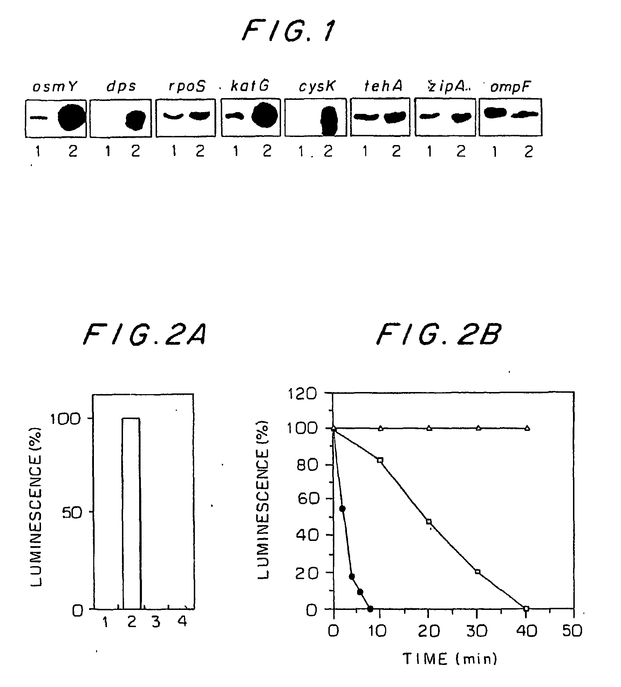

Figure 1 are gels showing the effect of DHCP on the levels of mRNAs. Total RNA was extracted by the hot phenol method as described in the Materials and Methods section of Example 1, and primer extension analysis was carried out with oligonucleotides corresponding to *osmY, dps, rpos, katG, cysK, tehA*, *zipA,* and *ompF.* Lanes 1 and 2 in each case represent mRNAs isolated from control (untreated) and DHCP treated cells, respectively.

Figures 2A and 2B are graphs showing DHCP inhibits AI-2 activity. The AI-2 activity assay was carried out as described in Materials and Methods section of Example 1. The luminescence seen with *V. harveyi* BB152 was assumed as 100% and the other activities were expressed as relative %. In Fig. 2A, the lanes are as follows: Sterile AB medium, lane 1; *V. harveyi* BB152 culture fluid, lane 2; DHCP (250 µM), lane 3; DHCP (250 µM) plus *V. harveyi* BB152 culture fluid, lane 4. In Fig. 2B, DHCP was added to the reaction mixtures after maximum luminescence was generated by AI-2, at a concentration of 250 µM (closed circles) and 100 µM (open squares). The corresponding control activity of AI-2 without DHCP addition is also shown (open triangles). The light production was monitored at various time points with a liquid scintillation counter.

### DETAILED DESCRIPTION OF THE INVENTION

The invention will be more readily understood through reference to the following example which is provided by way of illustration and is not intended to be limiting of the present invention.

### EXAMPLE 1

The present inventors analyzed the global transcriptional pattern of *E. coli* in response to DHCP by DNA microarray to (i) explore the manifestation of the antibacterial activity of DHCP and (ii) explore the possibility of DHCP being involved in the quorum-sensing pathways of *E. coli*. The results obtained by the present inventors showed that (i) DHCP has widespread effects in *E. coli*, affecting genes encoding proteins involved in general metabolism and membrane synthesis and functions, and that (ii) the genes comprising quorum-regulated processes such as virulence, motility and outer membrane functions are affected by DHCP treatment. The levels of gene expression related to virulence such as sapF (Lopez-Solanilla et al., 1998), potA, and potB (DeLisa et al., 2001) decreased in the cells treated with DHCP. The sapF gene from *E. coli* is homologous to sapF from *V. fischeri*, the latter having been shown to be enhanced by the LuxR-AI complex, and it was suggested that sapF to play a role in the bioluminescence of V. fischeri (Chen et al., 2000). In addition, cysK which is a known quorum sensing gene working in an alternate pathway(s) in *E. coli* (Baca-DeLancey et al., 1999) increases considerably in response to DHCP. The results, as presented in this Example, suggest that DHCP regulates the switching on/off of the different quorum-sensing circuits in *E. coli*.

### MATERIALS AND METHODS

### Bacterial strains

*E. coli* wild-type strain JM83 [F*⁻araΔ*(*lac-proAB) rpsL*(*str^{r}*)] (Yanisch-Perron et al., 1985) was grown in Luria broth (LB). *V. harveyi* strains BB152 and BB170 were a gift from Dr. Bonnie Bassler (Princeton University). These strains were grown in AB medium as described previously (Surette et al., 1999).

### RNA isolation

*E. coli* cells grown overnight in LB medium were diluted into fresh LB medium. After the growth reached a Klett unit of 50, DHCP was added (250 µM) and growth was further monitored. After growth reached a Klett unit of 90-100, it was diluted 10-fold into a medium containing the same concentration of DHCP as described previously (Phadtare et al., 2001). The cells were harvested after a total of 8 h of incubation with DHCP. Control cells were grown in a similar manner without DHCP and harvested at an OD₆₀₀ comparable to the final OD₆₀₀ of the DHCP-treated cells. The total RNA was extracted by the hot phenol method described previously (Sarmientos et al., 1983). It was further purified by RNeasy Minikit (Qiagen) and was then treated with DNase I followed by phenol-chloroform treatment and ethanol precipitation. It was quantified by measuring absorbance at 260 nm. The purity of RNA was confirmed by agarose gel electrophoresis.

### DNA microarray analysis

The mRNAs were labeled with Cy3-dUTP and Cy5-dUTP (Amersham Pharmacia). Random Hexamer pd(N)₆ (Amersham Pharmacia) was used as a primer and the IntelliGene *E. coli* CHIP Version 1 (Takara Shuzo, Co. Ltd., Japan) the DNA microarray was used. This represents the entire ORF of *E. coli* K-12 W3110.

### Primer extension

The primers used for detection of the respective mRNAs were: Primer 750077, 5'-TACAGCCAGCAGAGTTTTCGAAAT-3' (SEQ ID NO:1), that corresponds to the sequence from the 15^{th} to the 8^{th} codon of *osmY* (Yim et al., 1992); primer 750078, 5'-ATAAAGCAGATTGGTCGCTTTTGA-3' (SEQ ID NO:2), that corresponds to the sequence from the 16^{th} to the 9^{th} codon of dps (Altuvia et al., 1994); Primer 979747, 5'-CAGCGTATTCTGACTCATAAGGTG-3' (SEQ ID NO:3), that corresponds to the region from the 6^{th} codon of *rpoS* (Takayanagi et al., 1994); primer 956660, 5'-AGT GGCTGTGGTGTTATGGATATC-3' (SEQ ID NO:4), that corresponds to the region from 13^{th} to the 6^{th} codon of *katG* (Triggs-Raine et al., 1998); primer 3969805, 5'-CAGGCGAACCAGCGGCGTGTGACC-3' (SEQ ID NO:5), that corresponds to the region from 20^{th} to the 13^{th} codon of *cysK* (Byrne et al., 1988); primer 3969806, 5'-GTAGCCTGCCGGCAAATTGAGCAC-3' (SEQ ID NO:6), that corresponds to the region from 13^{th} to the 6^{th} codon of *tehA* (Walter et al., 1991); primer 3969807, 5'GATTAATATCAGACGCAAATCCTG-3' (SEQ ID NO:7), that corresponds to the region from 10^{th} to the 3^{rd} codon of *zipA* (Hale et al., 1997); and primer 7018 5'-ACGGGATCCTTCATCATTATTTATTA-3' (SEQ ID NO:8), that includes the region encompassing from the first codon of *ompF* (accession numbers, J01655, M10311 and M10312). The primers were labeled with [γ-³²P] ATP (Du-Pont-New England Nuclear] by using T4 polynucleotide kinase (Gibco BRL). Primer extension was carried out with 5 µg of RNA at 42 °C for 1 h in a final reaction volume of 10 µl, with 50 mM Tris-HCl (pH 8.5), 8 mM MgCl₂, 30 mM KCl, 1mM dithiothreitol, 0.4 pmol of ³²P-labeled primer, 0.5 mM each of the dNTPs, 10 U RNase inhibitor (Boehringer Mannheim) and 6.25 U of reverse transcriptase (Boehringer Mannheim). The products were analyzed on a 6% polyacrylamide gel under denaturing conditions. Quantitation of primer extension products was carried out by direct radioactive measurements.

### AI-2 assay

The AI-2 assays using *V. harveyi* reporter strain (BB170) were carried out as described by Surette and Bassler (Surette et al., 1999). Cell-free culture fluid from *V. harveyi* BB152 strain was used as a positive control. Sterile AB medium was used a negative control. Briefly, the *V. harveyi* BB170 strain was grown overnight with aeration at 30°C in the AB medium and the cells were then diluted 1:5000 in the fresh medium. The diluted culture (90 µl) was added in the assay mixture containing 10 µl of AB medium or cell-free culture fluid of *V. harveyi* BB152/DHCP (100-250µM). To check the effect of DHCP on the light production by AI-2, it was added to the reaction mixture after the maximum luminescence was achieved. The light production was monitored with a liquid scintillation counter.

### RESULTS

Previously, the laboratory of the present inventors have shown that the growth of *E. coli* JM83 was severely impaired in the presence of DHCP (250 µM) after 3 h of incubation and that cells stopped growing after 5 h (Phadtare et al., 2001). The main objective in the present study was to identify all of the *E. coli* ORFs that exhibit significant increase or decrease in mRNA abundance caused by the DHCP treatment. The cell densities of the control (untreated) and the DHCP-treated cells used were the same; thus, the changes seen in the microarray were not substantially influenced by the difference in cell densities. RNAs isolated from the respective cells were used to generate probes for hybridization of DNA arrays and the data were quantified as described in the Materials and Methods section of this Example. Calculation of the log expression ratios of the corresponding spots allowed pair-wise comparisons of the relative transcript levels for each of the *E. coli* genes under the two growth conditions. Only those genes whose expression levels differed by log ratio of at least four were considered. In some cases, genes belonging to same operon or category were considered even if the log ratios differed by the factor of only three or little less than three. Log ratios above zero value indicate induction and below zero value indicate repression by DHCP treatment. The results seen by DNA microarray analysis were confirmed by primer extension using oligonucleotides corresponding to some of the genes significantly affected. The results are shown in Fig. 1 and summarized in Table 1. The results of both methods are in agreement with each other.

**TABLE 1. Comparison of data from DNA microarray and primer extension**

| Gene | Microarray | Primer extension |
|---|---|---|
| *osmY* | 12.00 | 15.00 |
| *dps* | 13.00 | 12.00 |
| *rpoS* | 4.00 | 4.00 |
| *katG* | 9.00 | 9.00 |
| *cysK* | 20.00 | 18.00 |
| *tehA* | 5.00 | 5.00 |
| *zipA* | 4.50 | 5.00 |
| *ompF* | 0.06 | 0.06 |

| | | |
|---|---|---|
| Ratio of the respective RNA levels in the DHCP-treated to control (untreated) cells in each case is shown. | | |

The laboratory of the present inventors observed that a large number of the responding genes comprised broad functional categories: (i) genes encoding ribosomal proteins, (ii) genes encoding the global regulator of stress response-RpoS and the RpoS-regulated proteins, (iii) genes involved in membrane synthesis and functions such as transport, (iv) genes encoding proteins involved in general metabolism, (v) genes encoding proteins with miscellaneous functions, and (vi) genes encoding hypothetical proteins and proteins of unknown functions or class.

### Genes encoding ribosomal proteins are affected by DHCP treatment

As the concentration of DHCP chosen for the present experiment severely impaired the growth, one would expect significant secondary effects due to growth inhibition itself and this does not necessary reflect the site of action of DHCP. One of the manifestation of slower growth rate is that the translationary machinery of the cell is affected, evidenced by lower levels of ribosomal proteins. As seen from Table 2, most of the genes encoding ribosomal L proteins showed reduced levels, although in most cases the effect is not severe (2-3 times).

**TABLE 2. Effect of DHCP on genes encoding ribosomal proteins**

| Gene | Gene product and/or function | Ratio (DHCP-treated/control) |
|---|---|---|
| *rplA* | Ribosomal protein L1 | 0.23 |
| *rplB* | Ribosomal protein L2 | 0.25 |
| *rplC* | Ribosomal protein L3 | 0.28 |
| *rplD* | Ribosomal protein L4 | 0.27 |
| *rplE* | Ribosomal protein L5 | 0.34 |
| *rplF* | Ribosomal protein L6 | 0.30 |
| *rplI* | Ribosomal protein L9 | 0.25 |
| *rplJ* | Ribosomal protein L10 | 0.28 |
| *rplK* | Ribosomal protein L11 | 0.39 |
| *rplM* | Ribosomal protein L13 | 0.49 |
| *rplN* | Ribosomal protein L14 | 0.44 |
| *rplO* | Ribosomal protein L15 | 0.36 |
| *rplP* | Ribosomal protein L16 | 0.36 |
| *rplQ* | Ribosomal protein L17 | 0.47 |
| *rplR* | Ribosomal protein L18 | 0.38 |
| *rplS* | Ribosomal protein L19 | 0.35 |
| *rplV* | Ribosomal protein L22 | 0.28 |
| *rplW* | Ribosomal protein L23 | 0.27 |
| *rplX* | Ribosomal protein L24 | 0.31 |
| *rplY* | Ribosomal protein L25 | 0.28 |
| *rpmB* | Ribosomal protein L28 | 0.45 |
| *rpmE* | Ribosomal protein L31 | 0.39 |

### Effect of DHCP on membrane-associated functions

Next, the effect of DHCP on different classes of genes was examined. As seen from Table 3, 40 genes encoding proteins related to cell membrane were significantly affected. Predominantly, the cellular transport systems were affected especially those involving transport of iron (fecA, fecB, fecC, fecD, fepA and fepC) and spermidine/putrescine (potA, potB, potC and potD). The genes encoding outer membrane proteins (Omp) also decreased (5-15 times). The genes encoding proteins involved in motor action such as motA, motB and cheA decreased 4-6 times. Interestingly, atzN encoding zinc-transporting ATPase increased 11 times. The most prominent difference (47 times increase) was seen in the case of creD encoding CreD. Since the exact function of CreD is not known (Avison et al., 2001), it is difficult to judge the physiological significance of this observation. One interesting point is that creD is regulated by CreBC, the first member of the cre regulon and a presumed global regulator (Avision et al., 2001). Another gene regulated by CreBC is talA (Avision et al., 2001), which also increased 7.6 times in the present study (Table 6). talA encodes an enzyme involved in the mobilization of glyceraldehydes-3-phosphate into the pentose phosphate pathway. The other CreBC-regulated genes such as yids, and yieI, the products of which have not been assigned any function yet, also increased 4 times (Table 7). However the other genes known to be regulated by CreBC such as, ackA, pta, radC, malE and trgB were not induced by DHCP. In fact, ackA decreased 3.6 times (Table 5). Some additional factors may be involved in the regulation of expression of these genes.

The five-fold increase in the level of *tehA* encoding protein that confers resistance to tellurite seems to be significant. Tellurite causes toxicity in a number of gram-negative bacteria, the exact mechanism of this toxicity is not known, however it is shown that it causes oxidative stress and may replace sulfur in various proteins, rendering them nonfunctional (Garberg et al., 1999 and Summers et al., 1977). Overexpression of *tehA* also confers resistance to compounds such as tetraphenylarsonium CI, ethidium bromide, crystal violet and proflavin, similar to that inferred by multidrug resistance pumps (Turner et al., 1997).

**TABLE 3 Effect of DHCP on genes involved in membrane synthesis and membrane-associated functions**

| Gene | Gene product and/or function | Ratio (DHCP-treated/control) |
|---|---|---|
| *ansP* | L-asparagine permease | 0.29 |
| *atoE* | Short-chain fatty acids | 0.17 |
| *atzN* | Zinc-transporting ATPase | 11.30 |
| *cheA* | Chemotaxis protein CheA | 0.25 |
| *cpxP* | Periplasmic protein precursor | 4.95 |
| *creD* | Inner-membrane protein CreD | 47.00 |
| *czcD* | Cation efflux protein CzcD | 3.60 |
| *fadL* | Long-chain fatty acid transport protein | 0.10 |
| *fecA* | Iron(III) dicitrate transport protein FecA | 0.16 |
| *fecB* | Iron(III) dicitrate-binding protein | 0.15 |
| *fecC* | FecC protein | 0.21 |
| *fecD* | Iron(III) dicitrate transport system protein | 0.15 |
| *fecE* | Iron(III) dicitrate transport protein | 0.11 |
| *fepA* | Ferrienterochelin receptor precursor | 0.25 |
| *fepC* | Ferric enterobactin transport protein FepC | 0.40 |
| *glf* | UDP-galactopyranose mutase | 0.20 |
| *lysP* | Lysin-specific permease | 0.16 |
| *motA* | Chemotaxis protein MotA | 0.18 |
| *motB* | Chemotaxis protein MotB | 0.40 |
| *msyB* | Membrane protein | 4.25 |
| *nmpC* | Outer membrane porin protein precursor | 0.01 |
| *ompA* | Outer membrane protein a precursor | 0.23 |
| *ompC* | Outer membrane protein c precursor | 0.20 |
| *ompF* | Outer membrane protein f precursor | 0.06 |
| *ompT* | ProteinaseVII | 0.11 |
| *pheP* | phenylalanine transport protein pheP | 0.13 |
| *plsX* | PlsX protein | 0.28 |
| *potA* | Spermidine/putrescine transport protein A | 0.13 |
| *potB* | Spermidine/putrescine transport system | 0.13 |
| *potC* | Spermidine/putrescine transport system | 0.20 |
| *potD* | Spermidine/putrescine transport protein D | 0.19 |
| *rfc* | Probably O-antigen polymerase | 0.23 |
| *sdaC* | Probable serine transport protein | 0.25 |
| *secF* | Secretion protein SecF | 0.26 |
| *secG* | P12 cytoplasmic membrane protein | 0.30 |
| *secY* | SecY protein | 0.40 |
| *tehA* | Tellurite resistance protein TehA | 5.00 |
| *tsr* | Methyl-accepting chemotaxis protein1(mcp-1) | 0.22 |
| *tsx* | Nucleoside-specific channel-forming protein | 0.22 |
| *znu* | High-affinity zinc uptake system protein | 0.13 |

### DHCP induces RpoS and proteins regulated by RpoS

RpoS is a global stress response regulator and it is also known to be involved in quorum-sensing in *E. coli* (Hengge-Aronis, 2000 and Sitnikov et al., 1996). Next, the effect of DHCP on rpoS levels was examined and it was found that rpoS was induced 4 times after the treatment with DHCP. The gene dps encoding Dps, a protein induced by oxidative and osmotic stress (Altuvia et al., 1994 and Martinez et al., 1997) was induced 13 times (Table 4). Other genes such as osmY and katG encoding RpoS-regulated stress proteins OsmY and KatG, respectively (Hengge-Aronis, 2000 and Yim et al., 1994) were also significantly induced. Interestingly, genes encoding some of the other RpoS-regulated proteins that are not involved in stress response were also induced, probably as a manifestation of higher levels of RpoS itself. Examples of these include hdeB, otsA, poxB and wrbA (Table 4).

**TABLE 4 Effect of DHCP on genes involved in membrane synthesis and membrane-associated functions**

| Gene | Gene product and/or function | Ratio (DHCP-treated/control) |
|---|---|---|
| *dps* | DNA-binding protein Dps | 13.00 |
| *hdeB* | 10k-1 protein precursor | 6.00 |
| *katG* | Catalase HPI | 9.00 |
| *osmY* | Hyperosmotically induced protein | 12.00 |
| *otsA* | Tehalose-6-phosphate synthase | 11.00 |
| *poxB* | Pyruvate dehydrogenase | 15.00 |
| *rpoS* | RNP polymerase sigma factor RpoS | 4.00 |
| *wrbA* | Trp repressor binding protein | 14.00 |

### Effect of DHCP on the genes involved in the general metabolism

How the DHCP treatment affected the general metabolism of the cell was next examined. Similar to the effect on membrane, DHCP treatment affected a number of processes involved in general metabolism of the cell, some of these probably being secondary effects of the treatment. 44 genes showed a significantly different level of expression after DHCP treatment (Table 5). The most prominent genes were the ones encoding proteins involved in cysteine synthesis. A remarkable increase was observed in the genes belonging to the regulon involved in synthesis of Cys, such as *cysA, cysD, cysH, cysI, cysJ, cysK, cysM, cysN, cysP, cysQ, cysU,* and *cysW* (3-20 times). Among these, *cysK* showed the maximum increase of 20 times. Interestingly, *cysG* and *cysS,* which do not belong to this regulon, were not affected by the DHCP treatment. Genes belonging to other operons induced by the DHCP treatment were the ones belonging to the *gal* operon and the *moa* operon involve in the galactose and molybdenum metabolisms, respectively. On the other hand, the genes belonging to the *Ent* operon decreased. The *fru* (*fruB* and *fruK*) system was also severely inhibited (8 and 33 times, respectively).

**TABLE 5. Genes involved in general metabolism responding to DHCP**

| Gene | Gene product and/or function | Ratio (DHCP-treated/control) |
|---|---|---|
| *ackA* | Acetate kinase | 0.28 |
| *AldH* | Aldehyde dehydrogenase homolog | 6.50 |
| *CysA* | Sulfate/thiosulphate transport protein | 3.00 |
| *CysD* | Sulfate adenylyltranseferase | 5.88 |
| *CysH* | Adenylsulphate reductase | 6.50 |
| *Cysl* | Sulfite reductase(NADPH) alpha subunit | 7.30 |
| *CysJ* | Sulfite reductase(NADPH) beta subunit | 4.70 |
| *CysK* | Cysteine synthase | 20.00 |
| *CysM* | O-acetylserine (thiol)-lyase B | 3.00 |
| *CysN* | Sulfate adenylyltransferase | 5.00 |
| *CysP* | Thiosulfate-binding protein CysP precursor | 3.55 |
| *CysQ* | Ammonium transport protein CysQ | 3.28 |
| *cysU* | Sulfate transport system permease | 4.99 |
| *CysW* | Sulfate/thiosulfate transport protein CysW | 15.00 |
| *EntA* | 2,3-dihydro-2,3-dihydroxybenzoate | 0.44 |
| *EntB* | Isochorismatase | 0.23 |
| *EntC* | Isochorismate synthase | 0.45 |
| *EntE* | Enterochelin synthetase | 0.32 |
| *Fbp* | Fructose-1,6-biphosphatase | 4.00 |
| *FruB* | PTS system | 0.12 |
| *FruK* | I-phosphofructokinase | 0.03 |
| *Gale* | UDP-glucose 4-epimerase | 4.60 |
| *GalM* | Aldose 1-epimerase | 4.00 |
| *GalP* | Galactose proton symport | 5.21 |
| *GAlT* | Galactose-1-phosphate uridylyl transferase | 6.50 |
| *GlnH* | Glutamine-binding protein precursor | 4.90 |
| *GltB* | Glutamate synthase (NADPH) large chain | 3.50 |
| *Gsp* | Glutathionylspermidine synthase/amidase | 3.80 |
| *HmpA* | Flavohemoprotein | 3.80 |
| *HyfG* | Hydrogenase-4 component G | 10.00 |
| *IlvB* | Acetolactate synthase | 4.00 |
| *LysU* | Lysine-tRNA ligase | 4.00 |
| *MoaA* | Molybdenum cofactor biosynthesis protein | 3.55 |
| *MoaB* | MoaB protein | 4.00 |
| *MoaC* | -MoaC protein | 2.86 |
| *MoaD* | Molybdopterin (mpt) converting factor | 4.30 |
| *Ndk* | Nucleoside-diphosphate kinase | 0.20 |
| *PfkB* | 6-Phosphofructokinase isoenzyme | 4.91 |
| *PheS* | Phenylalanine-tRNA ligase | 0.25 |
| *PheT* | Phenylalanine-tRNA synthetase | 0.25 |
| *PtfB* | Phosphotransferase system enzyme II | 0.08 |
| *PyrD* | Dihdroordate oxidase | 0.22 |
| *rfbC* | dTDP-6-deoxy-D-glucose-3.5 epimerase | 0.21 |
| *RfbD* | dTDP-6-deoxy-L-mannose-dehydrogenase | 0.27 |

### DHCP affects number of proteins with diverse functions

Table 6 lists 35 genes showing significant level of induction or inhibition after DHCP treatment. Interestingly, *tehB* encoding TehB, involved in tellurite resistance was induced 5 times, similar to *tehA* (Table 3). *AhpF, bfr* and *soxS* encoding alkyl hydroperoxide reductase, bacterioferritin and SoxS protein respectively, that respond to oxidative stress (Agnez-Lima et al., 2001; Cha et al., 1995 and Chen et al., 1999) were also induced about 4 times. A gene (*mdaB*) encoding MdaB (modulator of drug activity) was also induced 10 times. Overproduction of MdaB imparts resistance to two topoisomerase inhibitors, Adriamycin and etoposide and presumably acts by modulating topoisomerase IV activity (Chatterjee et al., 1995). However, in the present study, levels of *parC* and *parE*, the genes encoding two subunits of topoisomerase IV did not change upon DHCP treatment. The gene *marA* encoding multiple antibiotic resistance protein MarA, was also induced 5 times. The genes *nrdH* and *nrdI* encoding glutaredoxin proteins were also significantly induced by DHCP. Interestingly, genes involved in virulence such as the *sap* (sensitivity to antimicrobial peptides) operon genes were inhibited by the DHCP treatment.

Table 7 lists the genes responding to DHCP that encode proteins to which any functions have not been yet assigned.

**TABLE 6 Effect of DHCP on genes encoding proteins with diverse functions**

| Gene | Gene product and/or function | Ratio (DHCP-treated/control) |
|---|---|---|
| *ahpF* | Alkyl hydroperoxide reductase | 3.50 |
| *Bfr* | Bacterioferritin | 4.60 |
| *CirA* | Colicin I receptor precursor | 0.16 |
| *CspA* | Cold shock protein | 0.38 |
| *CspB* | Cold shock protein | 0.38 |
| *CspF* | Cold shock protein | 0.14 |
| *Cspl* | Cold shock protein | 0.19 |
| *DnaJ* | DnaJ protein | 0.28 |
| *FlhC* | Flagellar transcriptional activator FIhC | 0.29 |
| *FlhD* | Flagellar transcriptional activator FIhD | 0.11 |
| *FliC* | Flagellin | 0.30 |
| *FliY* | FliY protein precursor | 9.00 |
| *Hns* | DNA-binding protein H-NS | 0.30 |
| *HsdS* | Type I restriction enzyme | 0.21 |
| *MarA* | Multiple antibiotic resistance protein MarA | 5.00 |
| *McrB* | 5-methylcytosine-specific restriction enzyme B | 0.10 |
| *McrC* | McrC protein | 0.11 |
| *MdaB* | Modulator of drug activity | 10.00 |
| *MrdA* | Penicillin binding protein | 0.25 |
| *MrdB* | Rod-shaped determining protein MrdB | 0.24 |
| *NemA* | N-ethylmaleimide reductase | 8.00 |
| *NrdH* | Glutaredoxin-like protein | 21.00 |
| *Nrdl* | Nrdl protein | 9.50 |
| *SapA* | Peptide transport periplasmic protein SapA | 0.28 |
| *SapC* | Peptide transport system permease protein | 0.28 |
| *SapD* | Peptide transport system ATP-binding protein | 0.17 |
| *SapF* | Peptide transport system ATP-binding protein | 0.24 |
| *Sfa* | Sfa protein | 0.24 |
| *SoxS* | SoxS protein | 4.34 |
| *Sped* | Adenosylmethionine decarboxylase | 0.23 |
| *Spot* | SpoT protein | 0.22 |
| *SuhB* | Extragenic suppressor protein SuhB | 0.23 |
| *TalA* | Patatire transaldolase | 7.60 |
| *TehB* | Tellurite resistance protein TehB | 5.00 |
| Z*ipA* | Cell division protein | 4.50 |

**TABLE 7 Effect of DHCP on genes coding proteins of unknown function or class**

| Gene | Ratio (DHCP-treated/control) |
|---|---|
| *yafB* | 6.5 |
| *YbgS* | 24 |
| *YdiC* | 11 |
| *YedU* | 5 |
| *YeeD* | 9 |
| *YggG* | *4* |
| *YhbW* | 4.5 |
| *YhcN* | 5.5 |
| *YhhQ* | 6.5 |
| *YhjX* | 9 |
| *YidS* | 4 |
| *yiel* | 4 |
| *yjbJ* | 8 |
| *YmdD* | 0.2 |
| *YmgA* | 13 |
| *YnhA* | 3.8 |
| *YnhC* | 7.5 |
| *YnhD* | 8.2 |
| *YnhE* | 10 |

### DHCP inhibits activity of AI-2

In the present study, the AI-2 assay designed by Suretter and Bassler (Surette et al., 1999) was used. The *V. harveyi* BB170 reporter strain has the quorum-sensing phenotype, sensor 1⁻, sensor 2⁺. It induces lux expression through the signaling system 2 detector. Addition of 10% cell-free culture fluid prepared from *V. harveyi* BB152 strain (containing the system 2 autoinducer) stimulates luminescence expression in the reporter strain. DHCP had been considered as a candidate for the autoinducer AI-2, involved in interspecies quorum-sensing but reported that DHCP did not have this activity (Schauder et al., 2001). The laboratory of the present inventors examined if DHCP inhibits the activity of AI-2. As seen from Fig. 2A consistent with previous report (Schauder et al., 2001), AI-2 isolated from *V. harveyi* BB152 strain showed luminescence (lane 2), while DHCP did not show AI-2 activity (lane 3). Sterile AB medium was used as a negative control (lane 1). When DHCP (250 µM- same concentration as the one used for DNA microarray analysis) was added to the assay immediately before addition of AI-2 containing cell-free extract, AI-2 activity was completely inhibited (lane 4). It has been reported that the AI-2 gave maximum luminescence at approximately 10 µM concentration (Schauder et al., 2001). It should be noted that the DHCP concentration used in the present assay was in excess of the AI-2 concentration. It was also tested if DHCP can inhibit AI-2 activity when it is added after maximum luminescence is generated by AI-2. As seen from Fig. 2B, when DHCP was added at a concentration of 250 µM (closed circles), AI-2 activity was reduced to zero within 8 min. When it was added at the concentration of 100 µM (open squares), the AI-2 activity was reduced to zero after 40 min. The corresponding control activity without DHCP addition (open triangles) was completely stable. The present inventors conclude that DHCP efficiently inhibits activity of AI-2.

### DISCUSSION

The results of the DNA array analysis of *E. coli* cells treated with DHCP are interesting from two aspects: the data indicate that DHCP causes widespread effects and is involved in the complex quorum-sensing network of *E. coli.*

### Antibacterial activity of DHCP

The present study shows that DHCP has global effects in *E. coli,* affecting many genes encoding proteins that are involved in general metabolism and membrane synthesis and function. Interestingly, a number of genes responding to oxidative and osmotic stress were upregulated. In addition, tehA, tehB and cysK that confer resistance to tellurite were upregulated significantly. The modes of resistance to tellurite and the mechanism of its toxicity have been of great interest to researchers and have not been fully understood. It has been shown that (i) cysK mediates tellurite resistance in *E. coli,* (ii) TehA and TehB confer resistance to tellurite; Cys residues in these proteins are involved in binding to tellurite and TehB needs S-adenosyl-methionine (SAM) to bind tellurite. It is a dimer that can bind both of these compounds in mediating resistance to tellurite, and (iii) tellurite generates oxidative stress and may replace sulfur in various proteins, rendering them nonfunctional (Syllick-Brenzinger et al., 2000; Garberg et al., 1999; Summers et al., 1977 and Vasquez et al., 2001). In the present study, in addition to *cysK, tehA* and *tehB*, the genes encoding proteins such as AhpF, Dps, KatG, SoxS, and Bfr, which respond to oxidative stress, were upregulated. This suggests that DHCP may be generating stress inside the cell and its manifestations are apparent in a number of secondary effects observed in the present study.

### Involvement of DHCP in quorum sensing

In gram-negative bacteria, quorum sensing typically involves an acylated homoserine lactone (HSL) autoinducer whose synthesis is dependent on a 'LuxI' autoinducer synthase and a cognate 'LuxR' autoinducer binding/transcriptional activator protein. Binding of HSL inducer by a LuxR protein results in the transcriptional activation of specific target genes. The LuxI-LuxR signaling cascade involved in the bioluminescence was first identified in *V. fisheri* (Hastings et al., 1977 and Nealson et al., 1979). This marine gram-negative bacterium regulates the expression of luciferase with this quorum-sensing circuit. On the other hand, in *V. harveyi,* two autoinducers, AI-1 and AI-2 for intra- and inter-species communication, respectively were identified (for a review see ref. Schauder et al., 2001). AI-2 is homoserine lactone and the exact structure of AI-2 is not known. Recently it has been proposed that AI-2 probably is a furanosyl borate diester (Chen et al., 2002). Shauder *et al* examined the quorum-sensing activity of DHCP and found that it did not have AI-2 function (Schauder et al., 2001). Interestingly, in the present study it is shown that DHCP in fact inhibited the activity of AI-2. In addition, DHCP may be generated by biosynthetic pathways involved in synthesis of AI-2. This raises an intriguing possibility that DHCP is involved in the regulation of AI-2 function and in turn may regulate AI-2-based quorum-sensing in *E. coli.*

Recently, DNA microarray analysis of the global transcriptional pattern of *E. coli* in response to AI-2 has been studied (DeLisa et al., 2001). Some of the observations include: (i) genes encoding proteins involved in motility such as MotA and MotB increased 3 times, (ii) the *potABCD* genes encoding proteins involved in spermidine/putrescine transport increased 3-5 times. These proteins show homology to proteins encoded by the att operon in plants known to be involved in virulence (Matthysse et al., 1996), (iii) genes encoding OmpA and OmpF increased 3.2 and 5.1 times, respectively. OmpA is presumably involved in the virulence of *Pasturella haemolytica* (Mahasreshti et al., 1997), and (iv) *cysK* and *rpoS* increased 3 and 1.2 times, respectively. Interestingly, with the DHCP treatment an opposite effect was seen, except for that on *cysK* and *rpoS* (Tables 4 and 5). This is consistent with the fact that DHCP inhibited the AI-2 activity in the reporter assay. It has also been shown that *cysK* is induced by a signal not similar to AI-2 in an alternate quorum-sensing pathway in *E. coli.* Unlike AI-2, this signal is produced by *E. coli* DH5α strain, independent of glucose and in stationary phase (Baca-DeLancey et al., 1999). However the genes supposedly induced by this signal such as *astD*, tnaB and *gabT* were not induced by DHCP. It is also shown that indole acts as a signal and induces *cysK,* but not *gabT,* suggesting the involvement of different quorum-sensing pathways (Wang et al., 2001). The present result may be similar to this. It is also important to note that the level of some of the genes affected by AI-2 was not changed upon DHCP treatment. Genes such as *rfa, wzb* or *hha* did not change significantly by DHCP treatment, but showed changes upon treatment with AI-2. The exact reason for this effect is not known, but it is possible that these genes are regulated by other factors in addition to AI-2 and mere inhibition of AI-2 activity does not lead to their downregulation. Recently, it has been reported that AI-2 from *S. typhimurium* controls the expression of an ABC transporter that functions in its uptake. This operon is of unknown function but is predicted to encode an ABC transporter complex with significant similarity to the ribosome transport operon (Rbs). This was named as the *lsr* operon (Taga et al., 2001). Although, this operon was not indicated in the DNA array analysis of the global effect of AI-2 (DeLisa et al., 2001) and also in the present studies, interestingly, AI-2 induced another operon encoding ABC transporter system, namely the pot operon (DeLisa et al., 2001). Consistently in our studies, DHCP treatment resulted in significant inhibition of this operon (Table 3). This suggests that AI-2 may be involved in the regulation of other ABC transport systems as well, and its inhibition by DHCP is reflected in the consequent inhibition of at least some of these operons.

The *sap* operon (*sapA* to *sapF*) has been shown to be involved in the virulence of *Erwinia chrysanthemi* (Lopez-Solanilla et al., 1998). It has been reported that the *sap* operon in *V. fisheri* is regulated by LuxR-AI complex and thus was presumed to play a role in the bioluminescence (Chen et al., 2000). The Sap proteins of *V. Fisheri* are homologous to Sap proteins from *E. coli.* DHCP inhibited the *sap* genes (Table 6), which is consistent with the inhibition of the bioluminescence in the reporter assay. A specific regulatory region-like sequence R&R* resides within the *sap* and the lux regulon of *V. fisheri.* LuxR-AI binds to this region to regulate respective gene expressions (Chen et al., 2000). It is possible that DHCP interferes with binding of LuxR-AI complex to the R&R* sequence and this in turn may inhibit the transcription of genes, such as the luciferase operon, in the reporter assay or the *sap* operon in *E. coli.* It is tempting to speculate that DHCP may 'turn off' one pathway of quorum-sensing involving AI-2 and 'turn on' the alternate pathway involving induction of *cysK.* Recently, it has been shown that the AI-2 activity decreased significantly following the induction of several plasmid-encoded genes in both low and high cell density cultures of *E. coli*. The AI-2 level was linearly related to the accumulation level of each protein product (DeLisa et al., 2001). This introduces the quorum-sensing pathway as a potential target for optimization strategies designed to improve recombinant product yield. The inhibition of AI-2 activity by DHCP may prove to be interesting in this aspect.

In conclusion, DHCP has widespread effects in *E. coli.* It inhibits some of the genes involved in quorum-sensing processes such as virulence and motility and inhibits activity of one of the quorum-sensing autoinducers itself. On the other hand, certain genes such as *cysK* known to be involved in alternate quorum sensing pathways were greatly induced, suggesting that DHCP may be regulating the switching on/off of the different quorum-sensing circuits in *E. coli.*

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications.

Reference to known method steps, conventional methods steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### REFERENCES

Agnez-Lima, L. F., P. Di Mascio, B. Demple, and C. F. Menck. 2001. Singlet molecular oxygen triggers the soxRS regulon of Escherichia coli. Biol Chem 382:1071-5.
Altuvia, S., M. Almiron, G. Huisman, R. Kolter, and G: Storz. 1994. The dps promoter is activated by OxyR during growth and by IHF and sigma S in stationary phase. Mol Microbiol 13:265-72.
Avison, M. B., R. E. Horton, T. R. Walsh, and P. M. Bennett. 2001. Escherichia coli CreBC is a global regulator of gene expression that responds to growth in minimal media. J Biol Chem 276:26955-61.
Baca-DeLancey, R. R., M. M. South, X. Ding, and P. N. Rather. 1999. Escherichia coli genes regulated by cell-to-cell signaling. Proc Natl Acad Sci U S A 96:4610-4.
Byrne, C. R., R. S. Monroe, K. A. Ward, and N. M. Kredich. 1988. DNA sequences of the cysK regions of Salmonella typhimurium and Escherichia coli and linkage of the cysK regions to ptsH. J Bacteriol 170:3150-7.
Cha, M. K., H. K. Kim, and I. H. Kim. 1995. Thioredoxin-linked "thiol peroxidase" from periplasmic space of Escherichia coli. J Biol Chem 270:28635-41.
Chatterjee, P. K., and N. L. Sternberg. 1995. A general genetic approach in Escherichia coli for determining the mechanism(s) of action of tumoricidal agents: application to DMP 840, a tumoricidal agent. Proc Natl Acad Sci U S A 92:8950-4.
Chen, C. Y., and S. A. More. 1999. Neisseria gonorrhoeae bacterioferritin: structural heterogeneity, involvement in iron storage and protection against oxidative stress. Microbiology 145 (Pt 10):2967-75.
Chen, H. Y., S. F. Weng, and J. W. Lin. 2000. Identification and analysis of the sap genes from Vibrio fischeri belonging to the ATP-binding cassette gene family required for peptide transport and resistance to antimicrobial peptides. Biochem Biophys Res Commun 269:743-8.
Chen, X., S. Schauder, N. Potier, A. Van Dorsselaer, I. Pelczer, B. L. Bassler, and F. M. Hughson. 2002. Structural identification of a bacterial quorum-sensing signal containing boron. Nature 415:545-9.
DeLisa, M. P., J. J. Valdes, and W. E. Bentley. 2001. Quorum signaling via AI-2 communicates the "Metabolic Burden" associated with heterologous protein production in Escherichia coli. Biotechnol Bioeng 75:439-50.
DeLisa, M. P., C. F. Wu, L. Wang, J. J. Valdes, and W. E. Bentley. 2001. DNA microarray-based identification of genes controlled by autoinducer 2-stimulated quorum sensing in Escherichia coli. J Bacteriol 183:5239-47.
Dyllick-Brenzinger, M., M. Liu, T. L. Winstone, D. E. Taylor, and R. J. Turner. 2000. The role of cysteine residues in tellurite resistance mediated by the TehAB determinant. Biochem Biophys Res Commun 277:394-400.
Garberg, P., L. Engman, V. Tolmachev, H. Lundqvist, R. G. Gerdes, and I. A. Cotgreave. 1999. Binding of tellurium to hepatocellular selenoproteins during incubation with inorganic tellurite: consequences for the activity of selenium-dependent glutathione peroxidase. Int J Biochem Cell Biol 31:291-301.
Hale, C. A., and P. A. de Boer. 1997. Direct binding of FtsZ to ZipA, an essential component of the septal ring structure that mediates cell division in E. coli. Cell 88:175-85.
Hastings, J. W., and K. H. Nealson. 1977. Bacterial bioluminescence. Annu Rev Microbiol 31:549-95.
Hengge-Aronis, R. 2000. Bacterial Stress Responses. ASM Press, Washington D.C. p 161-178.
Koyama, N. et al. 1999. Cyclopentanones, process for preparing the same, and the use thereof.
Lopez-Solanilla, E., F. Garcia-Olmedo, and P. Rodriguez-Palenzuela. 1998. Inactivation of the sapA to sapF locus of Erwinia chrysanthemi reveals common features in plant and animal bacterial pathogenesis. Plant Cell 10:917-24.
Mahasreshti, P. J., G. L. Murphy, J. H. Wyckoff, 3rd, S. Farmer, R. E. Hancock, and A. W. Confer. 1997. Purification and partial characterization of the OmpA family of proteins of Pasteurella haemolytica. Infect Immun 65:211-8.
Martinez, A., and R. Kolter. 1997. Protection of DNA during oxidative stress by the nonspecific DNA-binding protein Dps. J Bacteriol 179:5188-94.
Matthysse, A. G., H. A. Yarnall, and N. Young. 1996. Requirement for genes with homology to ABC transport systems for attachment and virulence of Agrobacterium tumefaciens. J Bacteriol 178:5302-8.
Miller, C. H., and J. A. Duerre. 1968. S-ribosylhomocysteine cleavage enzyme from Escherichia coli. J Biol Chem 243:92-7.
Miller, M. B., and B. L. Bassler. 2001. Quorum sensing in bacteria. Annu Rev Microbiol 55:165-99.
Nealson, K. H., and J. W. Hastings. 1979. Bacterial bioluminescence: its control and ecological significance. Microbiol Rev 43:496-518.
Phadtare, S., K. Yamanaka, I. Kato, and M. Inouye. 2001. Antibacterial activity of 4,5-dihydroxy-2-cyclopentan-1-one (DHCP) and cloning of a gene conferring DHCP resistance in Escherichia coli. J Mol Microbiol Biotechnol 3:461-5.
Sarmientos, P., J. E. Sylvester, S. Contente, and M. Cashel. 1983. Differential stringent control of the tandem E. coli ribosomal RNA promoters from the rrnA operon expressed in vivo in multicopy plasmids. Cell 32:1337-46.
Schauder, S., and B. L. Bassler. 2001. The languages of bacteria. Genes Dev 15:1468-80.
Schauder, S., K. Shokat, M. G. Surette, and B. L. Bassler. 2001. The LuxS family of bacterial autoinducers: biosynthesis of a novel quorum-sensing signal molecule. Mol Microbiol 41:463-76.
Sitnikov, D. M., J. B. Schineller, and T. O. Baldwin. 1996. Control of cell division in Escherichia coli: regulation of transcription of ftsQA involves both rpoS and SdiA-mediated autoinduction. Proc Natl Acad Sci U S A 93:336-41.
Summers, A. O., and G. A. Jacoby. 1977. Plasmid-determined resistance to tellurium compounds. J Bacteriol 129:276-81.
Surette, M. G., and B. L. Bassler. 1999. Regulation of autoinducer production in Salmonella typhimurium. Mol Microbiol 31:585-95.
Taga, M. E., J. L. Semmelhack, and B. L. Bassler. 2001. The LuxS-dependent autoinducer AI-2 controls the expression of an ABC transporter that functions in AI-2 uptake in Salmonella typhimurium. Mol Microbiol 42:777-93.
Takayanagi, Y., K. Tanaka, and H. Takahashi. 1994. Structure of the 5' upstream region and the regulation of the rpoS gene of Escherichia coli. Mol Gen Genet 243:525-31.
Triggs-Raine, B. L., B. W. Doble, M. R. Mulvey, P. A. Sorby, and P. C. Loewen. 1988. Nucleotide sequence of katG, encoding catalase HPI of Escherichia coli. J Bacteriol 170:4415-9.
Turner, R. J., D. E. Taylor, and J. H. Weiner. 1997. Expression of Escherichia coli TehA gives resistance to antiseptics and disinfectants similar to that conferred by multidrug resistance efflux pumps. Antimicrob Agents Chemother 41:440-4.
Vasquez, C. C., C. P. Saavedra, C. A. Loyola, M. A. Araya, and S. Pichuantes. 2001. The Product of the cysK Gene of Bacillus stearothermophilus V Mediates Potassium Tellurite Resistance in Escherichia coli. Curr Microbiol 43:418-23.
Walter, E. G., J. H. Weiner, and D. E. Taylor. 1991. Nucleotide sequence and overexpression of the tellurite-resistance determinant from the IncHII plasmid pHH1508a. Gene 101:1-7.
Wang, D., X. Ding, and P. N. Rather. 2001. Indole can act as an extracellular signal in Escherichia coli. J Bacteriol 183:4210-6.
Whitehead, N. A., A. M. Barnard, H. Slater, N. J. Simpson, and G. P. Salmond. 2001. Quorum-sensing in Gram-negative bacteria. FEMS Microbiol Rev 25:365-404.
Yanisch-Perron, C., J. Vieira, and J. Messing. 1985. Improved M13 phage cloning vectors and host strains: nucleotide sequences of the M13mp18 and pUC19 vectors. Gene 33:103-19.
Yim, H. H., R. L. Brems, and M. Villarejo. 1994. Molecular characterization of the promoter of osmY, an rpoS-dependent gene. J Bacteriol 176:100-7.
Yim, H. H., and M. Villarejo. 1992. osmY, a new hyperosmotically inducible gene, encodes a periplasmic protein in Escherichia coli. J Bacteriol 174:3637-44.

## Claims

1. Use of DHCP for enhancing the production of a recombinant polypeptide in a microorganism in vitro.

## Patentansprüche

1. Verwendung von DHCP zur Verstärkung der Produktion eines rekombinanten Polypeptids in einem Mikroorganismus *in vitro*.

## Revendications

1. Utilisation de DHCP pour stimuler la production d'un polypeptide recombinant dans un micro-organisme in vitro.
